# FASCICULE DE BREVET EUROPEEN

(11) **EP 2 364 667 B1**
(45) Date de publication et mention de la délivrance du brevet: **02.12.2015**
(21) Numéro de dépôt: 10194231.6
(22) Date de dépôt: 08.12.2010
(51) Int. Cl.: A61B 19/08

(54) **Champ operatoire sous-fessier notamment pour accouchement**
Gesässtuch insbesondere zur Entbindung
Underbottocks drape especially for labor

(30) Priorité: 11.03.2010 FR 1051766
(43) Date de publication de la demande: 14.09.2011
(73) Titulaire: Vygon, 95440 Ecouen (FR)
(72) Inventeur: Lestoquoy, Patrick, 59551, ATTICHES (FR); Carrez, Jean-Luc, 95440, ECOUEN (FR)
(74) Mandataire: Regimbeau

(56) Documents cités:
- US-A- 3 030 957
- US-A- 3 494 356
- US-A- 3 589 365
- US-A- 3 791 381
- US-A- 4 479 492
- US-A1- 2007 023 053

## Description

L'invention concerne d'une façon générale le matériel médical, et en l'espèce un champ sous-fessier adapté notamment pour l'accouchement.

Plus particulièrement, l'invention concerne un champ sous-fessier pour accouchement comportant une poche de recueil.

Les champs d'accouchement sont des feuilles souples généralement étanches, qui sont placées sur des tables opératoires afin de protéger la table d'une part, et pour le confort des femmes enceintes d'autre part.

Lorsque l'accouchement présente des complications, par exemple parce qu'une suture d'épisiotomie est nécessaire, la patiente est soulevée afin de retirer le champ utilisé pour l'accouchement et de le remplacer par un champ propre.

Néanmoins, cette opération de remplacement n'est pas très pratique à réaliser.

L'invention vise donc à proposer un champ modulable permettant d'être utilisé dans toutes les étapes d'un accouchement.

Pour cela, l'invention propose un champ opératoire sous-fessier, notamment pour accouchement, comprenant une feuille souple ayant une partie sous-fessière et une partie libre, comprenant un premier pagne sous-fessier fixé dans une partie supérieure de la partie sous-fessière et destiné à recevoir un patient durant l'opération, ainsi qu'un pagne de rechange, s'étendant sous le premier pagne sous-fessier, de sorte que durant l'opération, celui-ci soit protégé par le premier pagne sous-fessier.

Certains aspects préférés mais non limitatifs du champ selon l'invention sont les suivants :
- au moins une partie inférieure du pagne de rechange est protégée par le premier pagne sous-fessier ;
- une zone supérieure de la partie libre présente au moins une double épaisseur de feuille s'étendant transversalement, formant une cavité ouverte vers le bas en utilisation et dont un fond est fermé au moins au niveau des extrémités latérales de ladite cavité
- une extrémité supérieure du pagne de rechange est fixée de manière adjacente à la cavité ;
- le pagne de rechange est fixé dans une zone adjacente à la partie supérieure de la feuille sous-fessière ;
- une partie supérieure du premier pagne est réalisé dans un matériau absorbant ;
- une partie inférieure du premier pagne sous-fessier est réalisée dans un matériau imperméable ;
- le champ comprend en outre une poche de recueil disposée sur une face avant) de la feuille, ladite poche présentant une embouchure orientée vers une zone supérieure de la partie sous-fessière ;
- la poche est amovible ;
- l'embouchure de la poche comprend des moyens de fermeture ;
- la poche comprend en outre au moins une anse ;
- la poche est séparée en deux compartiments, de sorte qu'un premier des compartiments est adjacent à la face avant de la feuille lorsque la poche est disposée sur ladite face, et le deuxième compartiment s'étend parallèlement au premier compartiment, de l'autre côté du premier compartiment par rapport à la feuille ;
- le champ comprend en outre un deuxième pagne sous-fessier fixé sur la partie sous-fessière ;
- le premier et le deuxième pagne sous-fessier sont solidaires ; et
- le premier pagne sous-fessier est fixé au niveau de son bord supérieur dans la partie supérieure de la partie sous-fessière, et le deuxième pagne sous-fessier est fixé au niveau de ses bords latéraux sur le premier pagne sous-fessier.

Selon un deuxième aspect, l'invention propose l'utilisation d'un champ conforme à l'invention pour une opération, notamment au cours d'un accouchement, selon les étapes suivante :
- placer un champ sur une table opératoire de sorte que la poche soit adjacente au bord de table ;
- introduire une extrémité libre du pagne sous-fessier dans la poche ;
- le cas échéant, introduire une extrémité libre du pagne de rechange dans la poche ;
- asseoir la patiente sur le pagne sous-fessier ;
- exécuter l'opération ;
- retirer le pagne sous-fessier du champ ; et
- mettre le pagne de rechange en position.

Certains aspects préférés mais non limitatifs de l'utilisation du champ sont les suivants :
- la poche est détachée du champ préalablement à la mise en position du pagne de rechange ; et, en fonction de la position du pagne de rechange sur le champ,
- le champ est déplacé afin de positionner le pagne de rechange sous les fesses de la patiente.

Selon un troisième aspect, l'invention propose l'utilisation d'un champ conforme à l'invention pour une opération, notamment au cours d'un accouchement, selon les étapes suivantes :
- placer un champ sur une table opératoire de sorte que la poche soit adjacente au bord de table ;
- introduire une extrémité libre du pagne sous-fessier dans la poche ;
- asseoir la patiente sur le pagne sous-fessier ;
- exécuter l'opération ;
- retirer le pagne sous-fessier du champ ;
- retirer la poche du champ ; et, en fonction de la position du pagne de rechange sur le champ,
- déplacer le champ de manière à placer la partie libre du champ sous la patiente et mettre le pagne de rechange en position.

Certains aspects préférés mais non limitatifs de l'utilisation du champ sont les suivants :
- la poche est fermée lors de son détachement du champ ; et
- le pagne de rechange est étalé sur le champ préalablement à sa mise en place sur la table d'opération. Le document US 3 030 957 décrit un champ opératoire selon le préambule de la revendication 1.

D'autres caractéristiques, buts et avantages de la présente invention apparaîtront mieux à la lecture de la description détaillée qui va suivre, et en regard des dessins annexés donnés à titre d'exemples non limitatifs et sur lesquels :
La figure 1a est une vue en perspective d'une table opératoire sur laquelle est positionné un champ selon une première forme de réalisation, avant accouchement ;
La figure 1b est une vue latérale de la table et du champ de la figure 1a, avant accouchement ;
La figure 2a est une vue en perspective d'une table opératoire sur laquelle est positionné un champ selon une deuxième forme de réalisation, avant accouchement ;
La figure 2b est une vue latérale de la table et du champ de la figure 2a, avant accouchement ;
La figure 3 est une vue en perspective de la table opératoire et du champ des figures 1a et 1b, sans poche de recueil, avant repositionnement pour pratiquer notamment une suture d'épisiotomie ;
Les figures 4A à 4D représentent des vues en coupe de quatre formes de réalisation de feuilles pour un champ selon l'invention ;
La figure 5 une représentation tridimensionnelle d'une forme de réalisation d'une poche de recueil pouvant être utilisée avec un champ selon l'invention ;
La figure 6a est une vue en perspective d'une table opératoire sur laquelle est positionné un champ selon l'invention. avant accouchement ; et
La figure 6b est une vue latérale de la table et du champ de la figure 6a, avant accouchement.

Nous allons décrire à présent un champ conforme à la présente invention, dans son utilisation au cours d'une opération d'accouchement.

Néanmoins, le champ selon l'invention n'est pas limité dans son application aux accouchements, et est adapté pour la protection d'une table opératoire dans toute opération médicale ou chirurgicale dans laquelle il peut s'avérer nécessaire, au cours de la même opération, de changer le champ utilisé afin de poursuivre l'opération sur un champ propre.

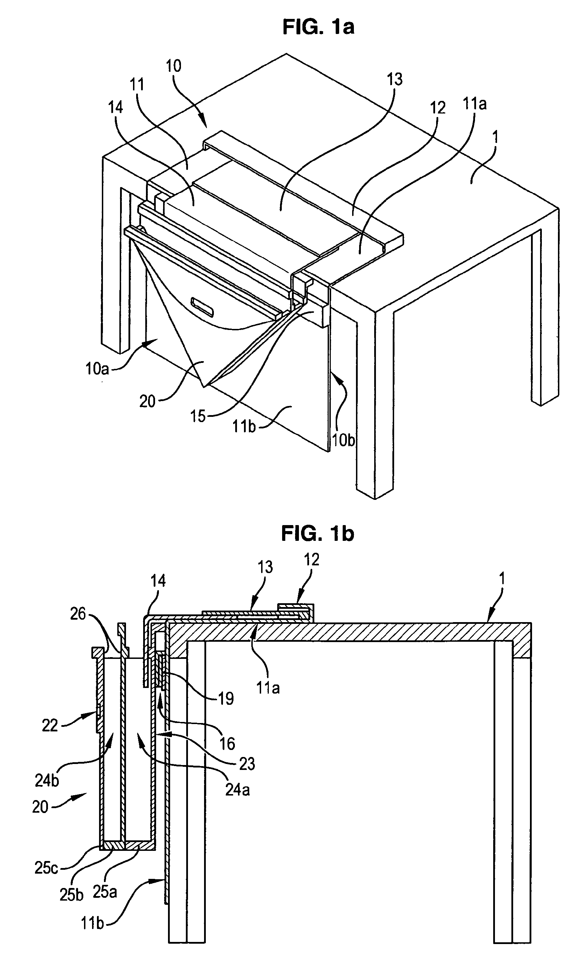

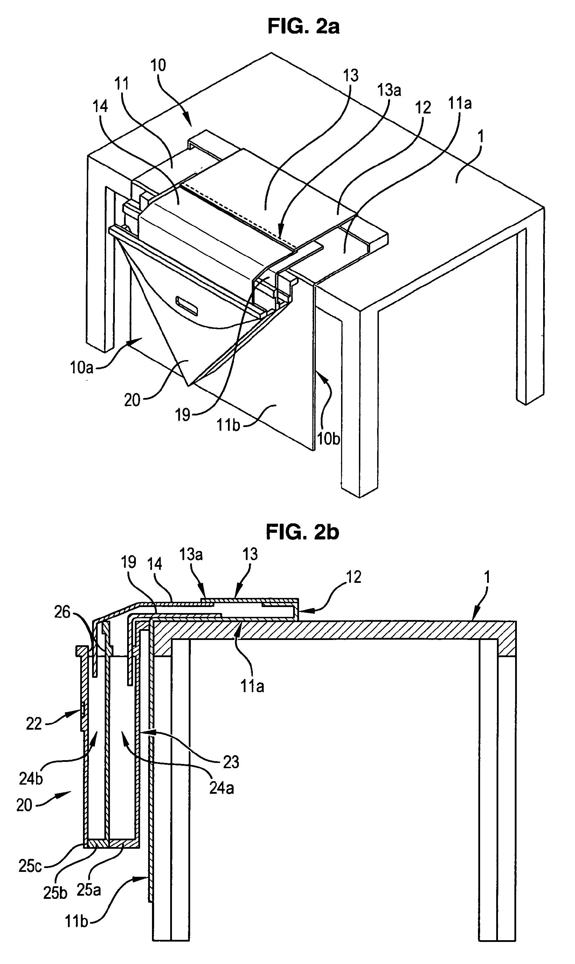

Comme illustré sur les figures 1 et 2, un champ sous-fessier 10 adapté pour l'accouchement comprend une feuille 11 ayant une partie sous-fessière 11 a et une partie libre 11 b.

La feuille 11 est ici en matière plastique, par exemple une résine de synthèse, ou tout autre matériau étanche adapté au domaine médical et chirurgical.

Selon une première forme de réalisation, l'extrémité libre de la partie sous-fessière 11a de la feuille 11 est repliée sur une face avant de la feuille 11 et fixée latéralement à la feuille pour constituer un rabat 12 sous lequel un opérateur peut glisser les mains pour mettre en place le champ 10 en le poussant sous les fesses de la patiente.

Le champ 10 comprend en outre un premier pagne sous-fessier 13, fixé dans une zone de la partie sous-fessière 11a adjacente au rabat 12.

Par exemple, un bord supérieur du premier pagne 13 est collé par thermoscellage ou par adhésion sur tout ou partie de la largeur du champ 10, sur ou sous la partie repliée du rabat 12, ou encore dans une zone adjacente au rabat 12.

Ce premier pagne sous-fessier 13 sert notamment pour le confort de la patiente lors de l'accouchement.

Le champ peut en outre comprendre un deuxième pagne sous-fessier 14, fixé sur la partie sous-fessière 11a.

Par exemple, et comme illustré sur les figures 1a et 1b, le premier pagne sous-fessier 13 peut être fixé sur le rabat 12, tandis que le deuxième pagne sous-fessier 14 peut être fixé dans une zone supérieure de la feuille sous-fessière 11 a adjacente au rabat 12 et s'étendre par-dessus le premier pagne sous-fessier 13.

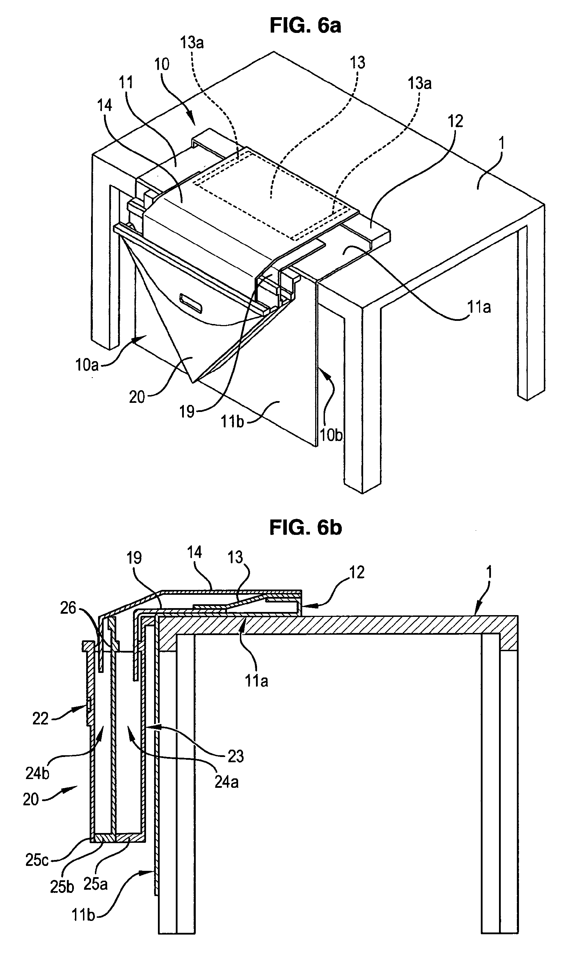

Le premier pagne sous-fessier 13 peut également être fixé sur le rabat 12 au niveau de ses bords latéraux et/ou de son bord supérieur, tandis que le deuxième pagne sous-fessier 14 peut être fixé au niveau de ses bords latéraux sur le pagne 13, comme illustré sur les figures 6a et 6b, par exemple par soudage d'une bande intermédiaire spécifiquement pelable 13a (constituée notamment d'au moins deux couches, dont une basse densité). Le deuxième pagne 14 peut alors présenter des dimensions plus grandes en largeur que celles du premier pagne 13, typiquement entre un et cinq centimètres, afin de faciliter sa fixation par-dessus le premier pagne 13. Dans cette variante de réalisation, le deuxième pagne 14 recouvre donc complètement le premier pagne 13 et le protège de toutes les projections de l'opération, tout en permettant de positionner le premier pagne 13 en partie supérieure du champ 1. Le premier pagne 13 est donc directement en place lors d'une remise au propre du champ 1.

En variante, le premier et le deuxième pagne sous-fessier 13 et 14 sont fixés ensemble de manière amovible au moyen d'un adhésif ou encore par soudage d'une bande intermédiaire spécifiquement pelable 13a, comme illustré sur les figures 2a et 2b, le premier pagne sous-fessier 13 étant en outre fixé dans une zone supérieure de la partie sous-fessière.

Le rôle du deuxième pagne sous-fessier 14 sera expliqué plus avant dans la suite de la description, en relation avec une poche de recueil 20.

Selon un mode de réalisation, le premier et/ou le deuxième pagne sous-fessier 13, 14 sont par exemple réalisés dans du non tissé, dans un assemblage non tissé, ou dans un voile de polyéthylène, c'est-à-dire dans des matériaux obtenus en assemblant entre elles des fibres par des procédés chimiques ou physiques autres que le tissage, bien connus par ailleurs de l'homme de l'art.

Avantageusement, le premier pagne sous-fessier 13 et, le cas échant, le deuxième pagne sous-fessier 14 sont réalisés dans un matériau absorbant au moins en partie supérieure, afin d'assurer le confort de la patiente, et imperméable en partie inférieure (il peut s'agir typiquement d'une doublure polyéthylène) afin de permettre le guidage de fluide provenant de l'opération vers un moyen de récupération, tel qu'une poche de recueil ou un seau ou encore de tout autre dispositif adapté pour un tel recueil.

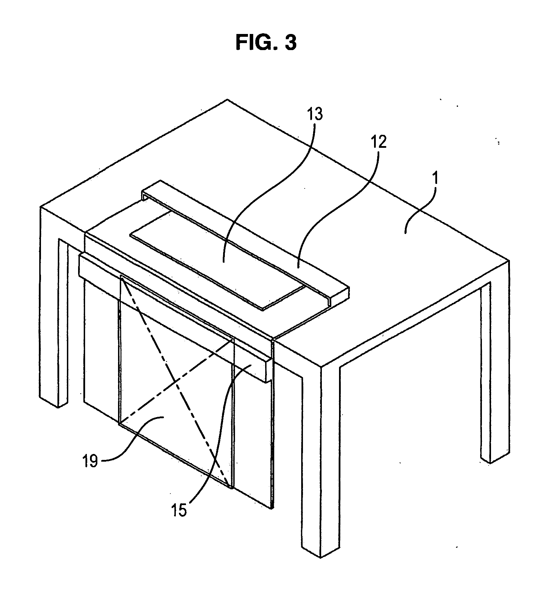

Dans les formes de réalisation décrites en relation avec les figures 1 à 4, une zone supérieure de la partie libre 11 b présente au moins une double épaisseur de feuille s'étendant transversalement, formant une cavité 16 ouverte vers le bas en utilisation et dont un fond 16a est fermé au moins au niveau des extrémités latérales de ladite cavité 16.

Cette configuration permet d'obtenir un deuxième rabat, dans une zone centrale du champ 10, dans lequel l'opérateur peut placer ses mains pour pousser et déplacer facilement le champ sur la table opératoire.

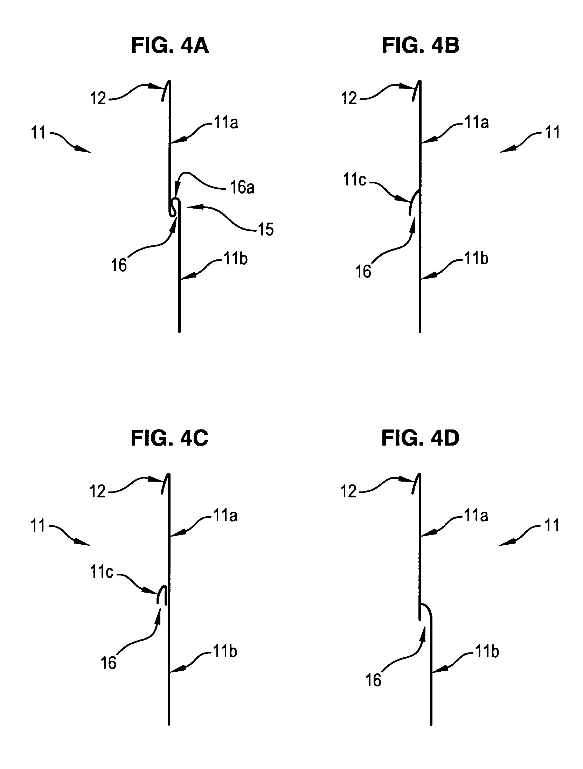

Dans une première variante de réalisation, illustrée sur la figure 4A, la zone supérieure de la partie libre 11b est repliée deux fois et fixée sur une face arrière 10b de la partie sous-fessière 11b (correspondant à la face arrière 10b de la feuille 11).

Ainsi, au niveau des plis 15 ainsi formés, la feuille 10 présente sur toute sa largeur une triple épaisseur.

Dans cette forme de réalisation, la feuille 11 est repliée de telle sorte que les plis 15 forment la cavité 16, le fond 16a de la cavité 16 étant fixé sur la face arrière 10b de la partie sous-fessière 11 b et fermé par l'un des plis 15.

Selon une deuxième forme de réalisation, la cavité 16 est formée à partir d'une feuille séparée 11 c qui est rapportée et fixée sur une zone inférieure de la partie sous-fessière 11a.

Par exemple, comme illustré sur la figure 4B, la feuille séparée 11 c est de largeur similaire à la feuille 10 du champ 10, et s'étend parallèlement à la feuille 11. Elle est alors fixée au niveau de son bord supérieur sur la face avant de la feuille 11, ainsi que sur ses bords latéraux.

En variante, comme illustré sur la figure 4C, la feuille séparée 11 c est pliée en deux dans le sens longitudinal de manière à former un U, de sorte que lorsqu'elle est rapportée sur la feuille 11, le champ présente une triple épaisseur de feuille.

Selon une autre variante encore, illustrée sur la figure 4D, la partie sous-fessière 11 a et la partie libre 11 b sont en fait deux feuilles indépendantes, rapportée et fixées l'une sur l'autre pour former la feuille 11.

Pour cela, un bord supérieur de la partie libre 11 b est fixée de manière décalée sur la face arrière d'une zone inférieure de la partie sous-fessière afin de former en outre la cavité 16.

Dans cette variante de réalisation, la feuille séparée 11 c est alors constituée par la partie libre 11 b de la feuille 11.

Quelle que soit la forme de réalisation de la cavité 16, ses bords latéraux sont en outre fixés sur les bords latéraux de la feuille 11, de manière à fermer latéralement la cavité 16.

Selon une première variante de réalisation, le champ 10 comprend en outre un pagne de rechange 19, qui peut également être en non tissé, fixé de préférence de manière adjacente à la cavité 16.

Un bord du pagne de rechange 19 est fixé dans le fond 16a de la cavité 16, sur tout ou partie de la largeur de la partie libre 11 b de la feuille 11, de sorte que le pagne de rechange 19 puisse être plié et rangé dans la cavité 16.

Ici, le pagne de rechange 19 est plié en accordéon puis placé dans la cavité 16, de telle manière que d'une part la cavité 16 retienne mécaniquement le pagne de rechange 19 en place, par exemple par friction, et d'autre part l'extrémité libre du pagne de rechange 19 soit aisément accessible par l'opérateur.

En variante, le pagne de rechange 19 est retenu dans la cavité par des moyens mécaniques, tels qu'une sangle de retenue, un adhésif, etc.

Ainsi, l'opérateur peut aisément sortir le pagne de rechange 19 de la cavité 16, en libérant les moyens mécaniques et/ou en tirant simplement sur son extrémité libre.

Selon une autre variante encore, il est possible de fixer le pagne de rechange 19 par-dessus les plis 15 formant la cavité (ou ne pas ranger le pagne 19 dans celle-ci lors de l'opération), et de sorte que le pagne s'étende le long de la partie libre 11 b de la feuille 11 sous le(s) pagne(s) sous-fessier(s) 13, 14.

Selon une deuxième variante de réalisation, illustrée sur les figures 2a, 2b, 6a et 6b, le pagne de rechange 19 peut simplement être fixé sur la partie sous-fessière 11a, et s'étendre sous le(s) pagne(s) sous-fessier(s) 13, 14 (voir figures 2a et 2b). De préférence, le pagne de rechange 19 est fixé de manière à être partiellement recouvert par le pagne 13.

La cavité 16 est alors optionnelle.

Bien entendu, il est également possible de munir le champ 10 de plus d'un pagne de rechange 19, en disposant par exemple un premier pagne de rechange au niveau de la zone supérieure de la partie sous-fessière 11a, et un pagne de rechange supplémentaire dans ou par dessus la cavité 16.

Par ailleurs, le deuxième pagne sous-fessier 14 est de préférence plus large de quelques millimètres voire quelques centimètres que le pagne de rechange 19 de manière à recouvrir l'intégralité du pagne 19 et ainsi mieux le protéger.

De manière optionnelle, le champ 10 comprend en outre une poche de recueil 20, étanche, et adaptée pour être fixée sur la face avant 10a de la feuille 11, par exemple dans une zone centrale du champ 10.

Une telle poche 20 peut être mise en oeuvre aussi bien sur un champ conventionnel que sur un champ conforme à la présente invention.

Dans le cadre d'un champ d'accouchement, la poche 20 est prévue pour recevoir des fluides ainsi que d'autres éléments provenant de l'opération d'accouchement, tels que les pertes sanguines, le placenta, etc. Son embouchure 20a est donc orientée vers le haut en utilisation de la partie sous-fessière 11a de la feuille 11, tandis que son fond 20b est dirigé vers le bas.

La poche 20 peut être amovible, et peut être séparée du champ 10 à tout moment.

Par exemple, tout ou partie d'une face 23 de la poche est fixée par thermoscellage d'un multicouche pelable en polyéthylène (qui peut être la matière du champ 11 ou de la face 23 de la poche), par une bande intermédiaire similaire à la bande liant les pagnes 13 et 14, ou encore au moyen d'adhésifs sur la feuille 11.

Pour faciliter sa manipulation, la poche 20 peut comporter en outre des poignées, telles que des anses 21, 22 prédécoupées, rapportées et fixées au niveau de son embouchure 20a.

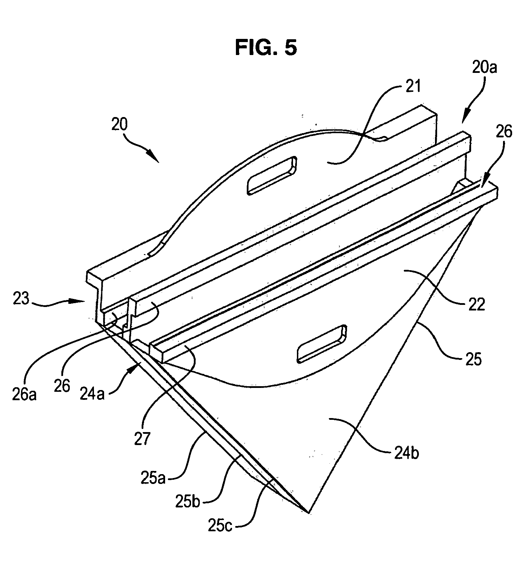

Par ailleurs, la poche 20 peut avoir toute forme appropriée, notamment une forme triangulaire, comme illustré sur la figure 5 ; l'embouchure 20a est alors pratiquée dans un côté du triangle, tandis que le fond 20b est formé par le sommet opposé audit côté.

La poche 20 est réalisée ici dans un film plastique transparent, et peut en outre comprendre, sur une face opposée à la face 23 qui est en regard du champ 10, des graduations permettant de mesurer un volume de fluides remplissant ladite poche 20.

La poche 20 peut comprendre en outre des moyens adhésifs 26 pour de fermer son embouchure 20a de manière étanche.

Par exemple, un adhésif double face protégé le cas échéant par une pellicule pelable peut être appliqué sur une périphérie de son embouchure 20a.

La poche 20 peut en outre comprendre un ourlet 27, parcourant tout ou partie de la périphérie de l'embouchure 20a, formant un canal dans lequel peut être placé un élément déformable 28 permettant de maintenir la poche 20 dans une position ouverte.

L'élément déformable 28 est par exemple un ruban métallique déformable, une bande plastique, etc.

Par ailleurs, la poche 20 peut être simple ou multiple, i.e. comporter un compartiment unique ou plusieurs compartiments.

Selon une forme de réalisation illustrée sur la figure 5, la poche 20 est double et comporte deux compartiments. Un premier compartiment 24a est adjacent à la face 23, tandis que le deuxième compartiment 24b, s'étend parallèlement au premier compartiment 24a, de l'autre côté par rapport à la face 23.

Pour cela, la poche 20 est formée par exemple de trois voiles triangulaires 25a, 25b, 25c, sensiblement identiques, soudés entre eux sur deux de leurs trois côtés. De la sorte, le voile 25a forme la paroi 23 de la poche 20, le voile 25c forme la paroi extérieure de la poche 20, sur laquelle peuvent être apposées les graduations, et le voile 25b constitue une paroi interne, délimitant les deux compartiments au sein de la poche 20.

Le sommet de chaque compartiment 21 a, 24b compris entre les deux côtés soudés forme alors le fond 20b respectif desdits compartiments, tandis que le côté laissé libre forme leur embouchure 20a.

Dans cette forme de réalisation, les anses 21 et 22 peuvent donc s'étendre à partir des voiles 25a, et 25b respectivement.

Avantageusement, le deuxième compartiment 24b de la poche 20 est fermé longitudinalement à mi-hauteur, par exemple par une soudure longitudinale des voiles 25b et 25c, de sorte que son fond ne soit pas accessible depuis l'embouchure. En effet, la partie adjacente à l'embouchure du compartiment 24b est destinée à recevoir des déchets solides (compresses usagées, etc.), tandis que sa partie inférieure, qui comporte les graduations, est destinée à permettre la lecture du niveau de liquide contenu par le premier compartiment 24a par transparence.

Par ailleurs, dans le cas de la poche à deux compartiments illustrée sur la figure 5, les adhésifs 26 sont appliqués sur les voiles 25a et 25b, au niveau de l'embouchure 20a et s'étendent sur toute la largeur des compartiments 24a, 24b, chacun étant en regard d'un autre adhésif prévu sur le voile 25b.

Ainsi, l'opérateur peut refermer l'un des compartiments indépendamment de l'autre compartiment.

De même, lorsque l'opérateur souhaite retirer la poche 20 du champ 10, il peut fermer les embouchures respectives des deux compartiments 24a et 24b en collant les voiles 25a, 25b, 25c deux à deux, ce qui permet en outre d'utiliser les anses 21, 22 de la poche 20 plus facilement.

Enfin, dans le cas d'une poche à deux compartiments, l'ourlet 27 peut être formé dans le voile 25c.

De la sorte, l'écartement du voile 25c par rapport au voile 25a permet, d'une part, d'ouvrir les deux compartiments 24b et 24a simultanément, puisque les voiles 25a, 25b, et 25c sont soudés sur leurs côtés latéraux, et, d'autre part, de maintenir l'ouverture de l'embouchure des compartiments 24a, 24b grâce à la déformation de l'élément déformable 28.

En variante, la poche comprend un deuxième ourlet formé dans le voile 25b et comprenant lui aussi un élément déformable.

Nous allons à présent décrire un exemple d'utilisation du champ 10 dans le cadre d'une opération d'accouchement.

Dans un premier temps, le champ 10 est placé sur une table opératoire 1. Pour cela, la partie sous-fessière 11a est placée à plat sur la table 1, de sorte que la partie libre 11 b pende vers le sol. L'opérateur peut par exemple placer ses mains dans le rabat 12 et pousser le champ 10 jusqu'à ce qu'il soit en position sur la table, sous les fesses de la patiente.

La poche 20 sur la feuille 11 est alors adjacente au bord de table 1.

De préférence, la poche 20 est fixée sur la feuille 11 de manière à recouvrir la cavité 16 (lorsque le champ en comporte une).

En variante, la poche 20 est fixée dans une partie sous-jacente à la cavité 16, dans la partie libre 11 b.

Le pagne sous-fessier 13 et/ou le pagne sous-fessier 14 est mis en place sous les fesses de la patiente pour l'opération.

Dans la suite de la description, on décrira la forme de réalisation dans laquelle le champ 10 comprend à la fois le pagne sous-fessier 13 et le pagne sous-fessier 14. Ceci n'est cependant pas limitatif.

Les premier et deuxième pagne 13 et 14 sont étalés sur le champ 10. Dans les formes de réalisation des figures 1 et 6, le premier pagne 13 est alors au moins partiellement protégé par le deuxième pagne 14.

L'opérateur agrandit alors l'embouchure 20a en déformant l'élément déformable 28, et introduit l'extrémité libre du deuxième pagne 14 dans la poche 20.

Dans le cas d'une poche à deux compartiments, l'opérateur introduit de préférence le pagne 14 dans le deuxième compartiment 24b.

Par ailleurs, si le pagne de rechange 19 est fixé dans la zone supérieure de la partie sous-fessière 11a, l'opérateur peut en outre ranger son extrémité libre dans le premier compartiment 24a. Il est alors protégé par les pagnes 13 et 14 qui s'étendent par-dessus lors de l'opération et le maintiennent propre.

En variante, dans le cas où le pagne 19 est fixé dans ou à proximité de la cavité 16, la poche 20 est fixée par-dessus le pagne de rechange 19 afin de le protéger et le maintenir propre durant l'opération.

La patiente peut alors s'asseoir sur les pagnes 13, 14, au niveau de la partie sous-fessière 11a.

Bien entendu, l'ordre de mise en place mentionné ci-dessus n'est aucunement limitatif.

Commence alors l'opération d'accouchement.

Comme précisé plus haut, le deuxième compartiment 24b sert à récupérer l'ensemble des déchets récupérés au cours l'accouchement, à l'exception du placenta. Cette récupération est favorisée par la mise en oeuvre de pagnes 14 et/ou 13 doublés de polyéthylène, dans la mesure où ils facilitent l'évacuation des fluides et des déchets de par leurs propriétés d'imperméabilité.

A la fin de l'accouchement, le pagne 14 est alors extrait du deuxième compartiment 24b puis placé dans le premier compartiment 24a.

Le deuxième compartiment 24b est alors éventuellement fermé grâce aux moyens adhésifs 26 pour éviter que son contenu se déverse.

Si des pertes sanguines surviennent suite à l'accouchement, elles sont alors collectées dans le premier compartiment 24a, et mesurées par transparence grâce aux graduations présentes sur le voile 25c du deuxième compartiment 24b.

L'opérateur peut alors contrôler aisément le volume de ces pertes sanguines, et déterminer si la patiente nécessite un traitement médicamenteux d'urgence (conforme par exemple au protocole Exadeli) afin de juguler l'hémorragie.

En variante, la poche 20 ne comprend qu'un seul compartiment. Les pertes sanguines sont alors récupérées dans le même compartiment que les autres déchets.

Après examen du placenta, la table opératoire est alors remise au propre par détachement du pagne 14, qui peut par exemple être placé avec le placenta dans le premier compartiment 24a.

Selon la première variante de réalisation, dans laquelle le pagne de rechange 19 est fixé dans la cavité 16 ou à proximité de celle-ci, le premier compartiment 24a est ensuite fermé grâce aux adhésifs 26, puis la poche 20 est détachée du champ 10 et évacuée de manière étanche.

Si suite à l'opération d'accouchement, il apparaît qu'une suture d'épisiotomie est nécessaire, l'opérateur sort le cas échéant le pagne de rechange 19 de la cavité 16 de sorte qu'il pende le long de la partie libre 11 b.

La patiente est alors soulevée, tandis que, simultanément, l'opérateur place ses mains dans la cavité ainsi vidée et pousse le champ 10, de manière à amener la partie libre 11b ainsi que le pagne propre 19 par-dessus la partie sous-fessière 11a, sous le corps de la patiente. La suture d'épisiotomie peut alors avoir lieu.

Selon les deuxième et troisième variantes de réalisation illustrées sur les figures 2a et 2b ainsi que 6a et 6b, dans laquelle l'une des extrémités du pagne de rechange 19 est fixée dans la zone supérieure du champ 10, l'opérateur sort simplement, le cas échéant, l'extrémité libre du pagne de rechange 19 de la poche 20 pour le mettre en place.

Selon le positionnement du pagne de rechange 19 par rapport au champ 10 (plus ou moins proche de la partie supérieure de la partie sous-fessière 11a), l'opérateur peut éventuellement translater le champ 10 en le poussant, comme expliqué plus haut, et le cas échéant retirer la poche 20.

La patiente est ensuite reposée sur le pagne 19, et le pagne sous-fessier 13 restant et la suture d'épisiotomie peut avoir lieu.

A la fin de l'opération d'épisiotomie, il est alors possible de retirer le pagne 19 et de le jeter, par exemple dans la poche 20 elle-même. La poche 20 peut alors être fermée grâce aux adhésifs 26, puis détachée du champ 10 et évacuée de manière étanche.

La patiente repose alors sur le pagne sous-fessier 13.

De la sorte, on garantit à tout moment l'utilisation d'une surface imperméable aux germes sur la table d'opération au moyen du champ sous-fessier, tout en permettant une évacuation étanche et ergonomique des déchets, du placenta, etc.

L'invention permet également de surveiller le niveau des pertes sanguines post-accouchement et d'assurer un nouveau champ propre pour une éventuelle suture d'épisiotomie avec des moyens de mise en place pratiques, ergonomiques et rapides.

## Revendications

1. Champ opératoire sous-fessier (10), notamment pour accouchement, comprenant une feuille souple (11) ayant une partie sous-fessière (11a) et une partie libre (11b), comprenant un premier pagne sous-fessier (13) fixé sur une partie supérieure de la partie sous-fessière (11) et destiné à recevoir un patient durant l'opération, et un deuxième pagne (14), s'étendant par-dessus le premier pagne sous-fessier (13), le deuxième pagne sous-fessier (14) recouvrant complètement le premier pagne sous-fessier (13), de sorte que durant l'opération, le premier pagne (13) soit protégé par le deuxième pagne sous-fessier (14),
**caractérisé en ce que** le deuxième pagne sous-fessier (14) est fixé au niveau de bords latéraux sur le premier pagne sous fessier (13) à l'aide d'une bande intermédiaire spécifiquement pelable comprenant au moins deux couches, dont une couche de basse densité.

2. Champ selon la revendication 1, **caractérisé en ce qu'**il comprend en outre un pagne de rechange (19), disposé sous le deuxième pagne sous-fessier (14).

3. Champ selon la revendication 2, dans lequel une zone supérieure de la partie libre (11 b) présente au moins une double épaisseur de feuille s'étendant transversalement, formant une cavité (16) ouverte vers le bas en utilisation et dont un fond (16a) est fermé au moins au niveau des extrémités latérales de ladite cavité (16).

4. Champ (10) selon la revendication 3, **caractérisé en ce qu'**une extrémité supérieure du pagne de rechange (19) est fixée de manière adjacente à la cavité (16a).

5. Champ (10) selon l'une des revendications 2 à 4, dans lequel le pagne de rechange (19) est fixé dans une zone adjacente à la partie supérieure de la feuille sous-fessière.

6. Champ (10) selon l'une des revendications 1 à 5, dans lequel une partie supérieure du premier pagne (13) est réalisée dans un matériau absorbant.

7. Champ (10) selon l'une des revendications 1 à 6, dans lequel une partie inférieure du premier pagne sous-fessier (13) est réalisée dans un matériau imperméable.

8. Champ (10) selon l'une des revendications 1 à 7, comprenant en outre une poche de recueil (20) disposée sur une face avant (10a) de la feuille (11), ladite poche (20) présentant une embouchure (20a) orientée vers une zone supérieure de la partie sous-fessière (11a).

9. Champ (10) selon la revendication 8, dans lequel la poche (20) est amovible.

10. Champ (10) selon l'une des revendications 8 ou 9, dans lequel l'embouchure (20a) de la poche (20) comprend des moyens de fermeture (26).

11. Champ (10) selon l'une des revendications 8 à 10, dans lequel la poche (20) comprend en outre au moins une anse (11, 12).

12. Champ (10) selon l'une des revendications 8 à 11, dans lequel la poche (20) est séparée en deux compartiments (24a, 24b), de sorte qu'un premier des compartiments (24a) est adjacent à la face avant (10a) de la feuille (10a) lorsque la poche (20) est disposée sur ladite face, et le deuxième compartiment (24b) s'étend parallèlement au premier compartiment (24a), de l'autre côté du premier compartiment (24a) par rapport à la feuille (10).

13. Champ (10) selon l'une des revendications 1 à 12, dans lequel le premier (14) et le deuxième (14) pagne sous-fessier sont solidaires.

14. Champ (10) selon l'une des revendications 1 à 13, dans lequel le premier pagne sous-fessier (13) est fixé au niveau de son bord supérieur dans la partie supérieure de la partie sous-fessière (11 a).

## Patentansprüche

1. Gesäßtuch (10), insbesondere für Entbindung, umfassend eine schmiegsamem Folie (11) mit einem Untergesäßteil (11a) und einem freien Teil (11b), umfassend einen ersten Untergesäßschurz (13), der an einem oberen Teil des Untergesäßteils (11) befestigt ist und zum Empfang eines Patienten während der Operation bestimmt ist, und einen zweiten Schurz (14), der sich über dem ersten Untergesäßschurz (13) erstreckt, wobei der zweite Untergesäßschurz (14) den ersten Untergesäßschurz (13) vollkommen bedeckt, so dass während der Operation der ersten Schurz (13) von dem zweiten Untergesäßschurz (14) geschützt ist,
**dadurch gekennzeichnet, dass** der zweite Untergesäßschurz (14) im Bereich von Seitenrändern auf dem ersten Untergesäßschurz (13) mit Hilfe eines speziell abziehbaren Zwischenbands befestigt ist, das mindestens zwei Schichten umfasst, davon eine Schicht mit geringer Dichte.

2. Tuch nach Anspruch 1, **dadurch gekennzeichnet, dass** es ferner einen Austauschschurz (19) umfasst, der unter dem zweiten Untergesäßschurz (14) angeordnet ist.

3. Tuch nach Anspruch 2, wobei eine obere Zone des freien Teils (11b) mindestens eine doppelte Foliendicke aufweist, die sich quer erstreckt, wobei ein in Benutzung nach unten offener Hohlraum (16) gebildet wird und von dem ein Boden (16a) mindestens im Bereich der seitlichen Enden des Hohlraums (16) geschlossen ist.

4. Tuch (10) nach Anspruch 3, **dadurch gekennzeichnet, dass** ein oberes Ende des Austauschschurzes (19) am Hohlraum (16a) angrenzend befestigt ist.

5. Tuch (10) nach einem der Ansprüche 2 bis 4, wobei der Austauschschurz (19) in einer Zone am oberen Teil der Untergesäßfolie angrenzend befestigt ist.

6. Tuch (10) nach einem der Ansprüche 1 bis 5, wobei ein oberer Teil des ersten Schurzes (13) aus einem absorbierenden Material hergestellt ist.

7. Tuch (10) nach einem der Ansprüche 1 bis 6, wobei ein unterer Teil des ersten Untergesäßschurzes (13) aus einem undurchlässigen Material hergestellt ist.

8. Tuch (10) nach einem der Ansprüche 1 bis 7, das ferner eine Sammeltasche (20) umfasst, die auf einer Vorderseite (10a) der Folie (11) angeordnet ist, wobei die Tasche (20) eine Mündung (20a) aufweist, die zu einer oberen Zone des Untergesäßteils (11a) zeigt.

9. Tuch (10) nach Anspruch 8, wobei die Tasche (20) lösbar ist.

10. Tuch (10) nach einem der Ansprüche 8 oder 9, wobei die Mündung (20a) der Tasche (20) Verschlussmittel (26) umfasst.

11. Tuch (10) nach einem der Ansprüche 8 bis 10, wobei die Tasche (20) ferner mindestens eine Ausbuchtung (11, 12) umfasst.

12. Tuch (10) nach einem der Ansprüche 8 bis 11, wobei die Tasche (20) in zwei Fächer (24a, 24b) getrennt ist, so dass ein erstes der Fächer (24a) an die Vorderseite (10a) der Folie (10a) angrenzt, wenn die Tasche (20) auf der Seite angeordnet ist, und sich das zweite Fach (24b) parallel zum ersten Fach (24a) auf der anderen Seite des ersten Fachs (24a) im Verhältnis zur Folie (10) erstreckt.

13. Tuch (10) nach einem der Ansprüche 1 bis 12, wobei der erste (14) und der zweite (14) Untergesäßschurz fest verbunden sind.

14. Tuch (10) nach einem der Ansprüche 1 bis 13, wobei der erste Untergesäßschurz (13) im Bereich seines oberen Rands im oberen Teil des Untergesäßteils (11a) befestigt ist.

## Claims

1. A sub-buttock operative field (10), notably for childbirth, comprising a flexible sheet (11) having a sub-buttock portion (11a) and a free portion (11b), comprising a first sub-buttock loincloth (13) attached on an upper portion of the sub-buttock portion (11) and intended to receive a patient during the operation, and a second loincloth (14) extending over the first sub-buttock loincloth (13), the second sub-buttock loincloth (14) completely covering the first sub-buttock loincloth (13), so that during the operation, the first loincloth (13) is protected by the second sub-buttock loincloth (14),
**characterized in that** the second sub-buttock loincloth (14) is attached at side edges on the first sub-buttock loincloth (13) by means of a specifically peelable intermediate strip comprising at least two layers, including a low density layer.

2. The field according to claim 1, **characterized in that** it further comprises a spare loincloth (19), positioned under the second sub-buttock loincloth (14).

3. The field according to claim 2, wherein an upper area of the free portion (11b) has at least one double sheet thickness extending transversely, forming a downward open cavity (16) during use and a bottom (16a) of which is closed at least at the side ends of said cavity (16).

4. The field (10) according to claim 3, **characterized in that** an upper end of the spare loincloth (19) is attached adjacent to the cavity (16a).

5. The field (10) according to one of claims 2 to 4, wherein the spare loincloth (19) is attached in an area adjacent to the upper portion of the sub-buttock sheet.

6. The field (10) according to one of claims 1 to 5, wherein an upper portion of the first loincloth (13) is made in an absorbent material.

7. The field (10) according to one of claims 1 to 6, wherein a lower portion of the first sub-buttock loincloth (13) is made in an impervious material.

8. The field (10) according to one of claims 1 to 7, further comprising a collecting pocket (20) positioned on a front face (10a) of the sheet (11), said pocket (20) having a mouth (20a) oriented towards an upper area of the sub-buttock portion (11a).

9. The field (10) according to claim 8, wherein the pocket (20) is removable.

10. The field (10) according to one of claims 8 or 9, wherein the mouth (20a) of the pocket (20) comprises closing means (26).

11. The field (10) according to one of claims 8 to 10, wherein the pocket (20) further comprises at least one handle (11, 12).

12. The field (10) according to one of claims 8 to 11, wherein the pocket (20) is separated into two compartments (24a, 24b), so that a first of the compartments (24a) is adjacent to the front face (10a) of the sheet (10a) when the pocket (20) is positioned on said face, and the second compartment (24b) extends parallel to the first compartment (24a), on the other side of the first compartment (24a) with respect to the sheet (10).

13. The field (10) according to one of claims 1 to 12, wherein the first (14) and second (14) sub-buttock loincloth are secured to each other.

14. The field (10) according to one of claims 1 to 13, wherein the first sub-buttock loincloth (13) is attached at its upper edge in the upper portion of the sub-buttock portion (11a).
